# EUROPEAN PATENT APPLICATION

(11) **EP 1 288 196 A1**
(43) Date of publication of application: **05.03.2003**
(21) Application number: 02255765.6
(22) Date of filing: 19.08.2002
(51) Int. Cl.: C07C 277/08

(54) **Process for the preparation of organo guanidinium salts**

(30) Priority: 31.08.2001 GB 0121163
(71) Applicant: Chordip Ltd., Billingham TS23 4HN (GB)
(72) Inventor: Atkinson, Brian, Brookfield, Middlesbrough, TS5 8HR (US); Lebedev, Victor, Moscow (RU)
(74) Representative: Gambell, Derek

(57) **Abstract**

A process for the preparation of monovalent or divalent organo guanidinium salts is described. If the product is a monovalent organo guanidinium salt, the process comprises mixing together a halohydrocarbon, an inorganic base, and a salt of unsubstituted guanidine. If the product is a divalent organo guanidinium compound, the process comprises mixing together a first halohydrocarbon, an inorganic base, and a salt of unsubstituted guanidine, followed by mixing of a second halohydrocarbon with the mixture so formed; this is followed by the mixing of a third halohydrocarbon with the mixture formed from the mixing of the second halohydrocarbon. Both processes do not require the absence of water; the use of an organic solvent is optional.

## Description

### TECHNICAL FIELD

This invention relates to halogen exchange reactions involving haloaromatic compounds and alkali metal fluorides, and more particularly to improved processes for producing polyfluorinated aromatics by catalyzed halogen exchange reactions.

### BACKGROUND

Halogen exchange reactions for fluorinating haloaromatic compounds using alkali metal fluorides have been extensively studied heretofore. Typically they involve the reaction of a chloroaromatic compound with potassium fluoride, rubidium fluoride or cesium fluoride by heating the reactants to extremely high temperatures (above about 400°C) in the absence of an ancillary diluent or solvent, or by conducting the reaction at temperatures of around 200-230°C in an aprotic solvent such as sulfolane. It has also been reported that organic fluorine compounds such as pentafluorobenzonitrile, tetrafluorophthalonitriles and pentafluoropyridine can be formed by reacting a corresponding chloro- or bromo-substituted compound with alkali metal halide such as potassium fluoride in benzonitrile as solvent at 190°C to 400°C in a sealed autoclave under autogenous pressure.

Use of catalysts in some exchange reactions has also been studied. Such catalysts have included quaternary ammonium salts, metal carbonyls, crown ethers and cryptates.

In most cases, the halogen exchange reaction is sluggish and tends to form product mixtures in which yields of polyfluorinated aromatics are relatively low, especially if the haloaromatic compound used is a polyhaloaromatic compound free from activating functionality such as nitro or carbonyl. For example, with hexachlorobenzene and potassium fluoride, typical product mixtures contain a mixture of co-products including hexafluorobenzene together with various chlorofluorobenzenes.

U.S. Pat. No. 5,824,827 describes a commercially feasible process whereby the halogen exchange reaction as applied to a wide variety of haloaromatic compounds may be conducted in large scale reaction equipment under relatively mild reaction conditions while providing commercially acceptable yields of the desired products. These improvements are achieved, *inter alia*, by use of an aminophosphonium catalyst such as for example tetrakis(diethylamino)phosphonium bromide.

It would be of considerable advantage if still another way could be found that would make it possible to conduct the halogen exchange reaction in a commercially feasible manner so that a wide variety of haloaromatic compounds could be produced in large scale reaction equipment under relatively mild reaction conditions while providing the desired products in commercially acceptable yields at least comparable to, if not better than, the yields achievable by use of aminophosphonium catalysts such as for example tetrakis(diethylamino)phosphonium bromide. This invention is deemed to fulfill this objective.

### SUMMARY OF THE INVENTION

This invention provides a new catalytic halogen exchange reaction using an alkali metal fluoride as the fluorine source. The process enables production of a wide variety of fluorinated aromatic compounds under relatively mild reaction conditions. Moreover, the process is applicable to use as starting materials of haloaromatic compounds containing one or more halogen atoms other than fluorine, including compounds which are devoid of activating groups, as well as compounds which possess one or more activating groups in the molecule. In fact, the process is especially well adapted for polyfluorination of perhaloaromatic compounds such as hexachlorobenzene, hexabromobenzene, pentachlorofluorobenzene, tetrachlorodifluorobenzene, trichlorotrilluorobenzene, dichlorotetrafluorobenzene, *etc.,* which have no activating group in the molecule. In addition, the catalyzed process can be conducted with smaller excesses of the alkali metal fluoride than generally required in prior processes.

The substantial improvements made possible by this invention are brought about at least in part by use of an organo guanidinium salt as catalyst in the process. The organo guanidinium salt has the advantage of being thermally stable under the halogen exchange conditions. As an example of such improvements, comparative reactions were conducted on a 2-liter scale. In these reactions hexachlorobenzene (0.86 mole) and potassium fluoride (6.0 moles) were heated in the presence of either the organo guanidinium salt, hexaethyl guanidinium bromide, as catalyst pursuant to this invention, or the aminophosphonium catalyst, tetrakis(diethylamino)phosphonium bromide. The catalysts were used at the level of 0.2 mole per mole of potassium fluoride. Nitrobenzene was employed as solvent in the amount of 50% wt/wt of the total reaction mixture. The following results were obtained after 12 hours 215°C:

**Table 1**

| | Hexaethylguanidinium Bromide | Tetrakis(diethylamino)phosphonium Bromide |
|---|---|---|
| C-Cl bond conversion | 93% | 91% |
| Molar yield of Fluorinated Products | 76.9% | 69.7% |
| Polymeric by-products | 35g | 68g |

Thus, in accordance with this invention there is provided in one of its embodiments a halogen exchange process which comprises heating an essentially anhydrous agitated mixture formed from ingredients comprising (I) at least one finely-divided essentially anhydrous alkali metal fluoride, (ii) at least one haloaromatic compound having on an aromatic ring at least one halogen atom of atomic number greater than 9, and (iii) an organo guanidinium salt catalyst, at one or more reaction temperatures at which at least one said halogen atom of said haloaromatic compound is replaced by a fluorine atom.

In a preferred embodiment of this invention, the process is conducted using as the initial haloaromatic compound(s) for the halogen exchange, at least one haloaromatic compound that is devoid of any activating functional group on the aromatic ring to which the halogen atom of atomic number greater than 9 is bonded.

A particularly preferred embodiment involves using as the initial haloaromatic ingredient to be subjected to the halogen exchange processing, one or more haloaromatic compounds that are not only devoid of any activating functional group on the aromatic ring to which the halogen atom of atomic number greater than 9 is bonded, but in addition either have no hydrogen atom on that aromatic ring, or have at most one hydrogen atom on the ring. Especially preferred haloaromatic compounds of this type are perhaloaromatic compounds of the formula C₆ClₙBrₘFₚ where n is from 0 to 6, m is from 0 to 6 and p is from 0 to 5, and where the sum of n, m and p is 6. Compounds in which m is zero have been used with outstanding success.

Another preferred embodiment includes conducting the process of this invention such that the essentially anhydrous agitated mixture when heated to one or more reaction temperatures is predominately a mixture of solids dispersed in a continuous liquid phase. Operations wherein the continuous liquid phase comprises at least one halogen-free, polar, anhydrous aprotic solvent constitute additional preferred embodiments of this invention.

Preferred catalyst ingredients for use in the various process embodiments of this invention are hexahydrocarbyl guanidinium salts, especially the halide salts.

A novel and very useful process for producing such hexahydrocarbyl guanidinium salts constitutes still another embodiment of this invention.

These and other embodiments, features and advantages of this invention will be further apparent from the ensuing description and appended claims.

### FURTHER DESCRIPTION OF THE INVENTION

The basic feed materials to the process of this invention are one or more haloaromatic compounds containing one or more aryl halogen atoms other than fluorine, alkali metal fluoride(s) of one or more alkali metals other than lithium (preferably alkali metal of atomic number 19 or above), and one or more organo guanidinium salt catalysts. Use of one or more ancillary solvents or diluents is optional, but preferable.

### Haloaromatic Ingredient

Any aromatic compound that has at least one replaceable halogen atom other than fluorine on the aromatic ring is a candidate ingredient for use in the process. The compound may have a homocyclic aromatic nucleus (i.e., at least one benzene ring system) or a heteroaromatic ring system. Also, the compound may contain one or more activating groups such as nitro, nitrous, carbonyl, cyano, sulfonic acid, etc., or it may be devoid of any such group. The compound contains one or more chlorine, bromine or iodine atoms, or any combination of Cl, Br, and/or I atoms on the aromatic ring and may also have one or more such halogen atoms on one or more side chains and/or on one or more non-aromatic homocyclic or heterocyclic rings bonded or fused to the aromatic ring system. In addition the compound may contain one or more fluorine atoms anywhere in the molecule including one or more aryl fluorine atoms provided the compound has at least one aromatic ring that contains at least one replaceable aryl halogen atom other than fluorine. The heteroatom in the halo-substituted aromatic ring where the fluorine substitution is desired is from 1 to 3 nitrogen atoms (e.g., the compound is, or has at least the ring system of, an aryl-halopyridine, an aryl-halopyridazine, an arylhalopyrimidine, an aryl-halopyrazine, an aryl-halotriazine where at least one aryl halogen atom is other than a fluorine atom). Other heteroatoms which can be present in side chains or additional ring systems of the compound include one or more nitrogen, oxygen, sulfur, phosphorus, boron or silicon atoms, or combinations of two or more of these. Generally speaking, the haloaromatic ingredient may contain in the range of up to 50 carbon atoms in the molecule, and preferably contains in the range of up to 20 carbon atoms in the molecule.

Preferred are haloaromatic compounds that are devoid of any activating group(s) in the molecule, as these usually undergo a halogen exchange reaction much less readily than their counterparts which have activating functionality in the molecule, and yet in the practice of this invention react much more readily.

Haloaromatic compounds with a heterocyclic ring system which are devoid of any activating group(s) in the molecule are preferred haloaromatic compounds. Preferably, the heterocyclic system has one heteroatom, and more preferably, the heteroatom is nitrogen. Most preferably, the heterocyclic ring moiety is pyridine.

Homocyclic haloaromatics are also preferred ingredients. As noted above, haloaromatics that are devoid of any activating functional group on the aromatic ring to which the halogen atom of atomic number greater than 9 is bonded and in addition, are devoid of any hydrogen atom on that aromatic ring constitute a preferred category of haloaromatic ingredient or feed material for the process. Especially preferred haloaromatic compounds of this type are perhaloaromatic compounds of the formula C₆ClₙBrₘFₚ where n is from 0 to 6, m is from 0 to 6 and p is from 0 to 5, and where the sum of n, m and p is 6. Compounds in which m is zero are especially desirable ingredients because of good reactivity in the process and generally lower cost. Moreover, there is a particularly pressing present need for methods for effectively producing polyfluorobenzenes, especially chloropentafluorobenzene and hexafluorobenzene, from their polychloro analogs such as hexachlorobenzene, pentachlorofluorobenzene, tetrachlorodifluorobenzene, trichlorotrifluorobenzene, or dichlorotetrafluorobenzene, or mixtures of any two or more of these, a need fulfilled by this invention.

Also fulfilled by this invention is the need for a method for effectively producing bromopentafluorobenzene from its polybromo analogs such as hexabromobenzene, pen-tabromofluorobenzene, tetrabromodifluorobenzene, tribromotrifluorobenzene, or dibromo-tetrafluorobenzene, or mixtures of any two or more of these.

Other haloaromatic compounds which can be converted into arylfluorinated compounds by use of this invention include, for example, mono-, di-, tri-, tetra- and pentachlorobenzenes, and bromo and iodo analogs thereof; mono and polychloro, bromo and iodo naphthalenes, tetrahydronaphthalenes, acenaphthalenes, biphenyls and terphenyls; alkyl- and haloalkyl-substituted analogs of the foregoing; chloro, bromo and iodo diarylethers and monoalkylmonoaryl ethers; 2-chloronitrobenzene; 4-chloronitrobenzene; 2,4-dinitrochlorobenzene; 3,4-dichloronitrobenzene; 3-chloro-4-fluoronitrobenzene; 2,4,6-trichloropyrimidine; tetrachloropyrimidine; 2-chloro benzonitrile; 4-chloro benzonitrile; pentachlorobenzonitrile; tetrachloroisophthalonitrile; 2-chloropyridine; 2,5-dichloropyridine; pentachloropyridine; 4-chlorophthalic anhydride; and still other similar compounds, such as are referred to in U.S. Pat. No. 4,684,734 to Kaieda, et al.

### Alkali Metal Fluoride Ingredient

Potassium fluoride, rubidium fluoride, and cesium fluoride are the preferred alkali metal halides used in the practice of this invention because of their higher reactivity in the exchange reaction. However, sodium fluoride can be used, especially where the haloaromatic ingredient has activating functionality on the haloaromatic ring, and in cases where only partial replacement of aryl chloride, aryl bromide or aryl iodide is desired.

Combinations of any two or more of the alkali metal fluorides can be used, including combinations in which lithium fluoride is present. Thus, mixtures of potassium fluoride, rubidium fluoride and/or cesium fluoride together with sodium fluoride or lithium fluoride, or both, can also be used if desired, although this is not recommended. To enhance its reactivity, the alkali metal fluoride should be in finely-divided or powdery anhydrous form. Potassium fluoride is the preferred fluorinating agent as it is the most cost effective reagent. One convenient way of ensuring that the fluorinating agent is suitably anhydrous is to form a slurry of the fluoride salt in a suitable volatile hydrocarbon such as benzene that forms an azeotrope with water, and heat the mixture to dryness, while of course suitably handling and disposing of the vapors. Another useful form of potassium fluoride for use in the process is the active form of KF produced using the procedure described by T. P. Smyth, A. Carey and B. K. Hodnett in *Tetrahedron,* Volume 51, No. 22, pp. 6363-6376 (1995). In brief, the procedure involves recrystallizing KF from a methanol solution by slow evaporation of the solvent, followed by drying at 100°C. Still another useful form of potassium fluoride is KF dispersed on CaF₂. This material is described by J. H. Clark, A. J. Hyde and D. K. Smith in *J. Chem. Soc. Chem. Commun*, **1986**, 791. Other activated forms of KF such as spray dried KF (N. Ishihara, et al. *Chem. Lett.,* **1981,** 761), and freeze dried KF (Y. Kimura, et al. *Tetrahedron Letters,* **1989,** 1271) can be used. It is also deemed possible to apply one or more of the foregoing activating procedures to other alkali metal fluorides such as cesium fluoride and/or sodium fluoride.

The proportions of alkali metal fluoride to the haloaromatic ingredient(s) being used can be varied. In theory there is no upper limit on the amount of alkali metal fluoride used relative to the amount of haloaromatic compound(s) used. If a very large excess of alkali metal fluoride is used relative to the amount of replaceable halogen present in the haloaromatic ingredient(s) present, the latter becomes the limiting reactant and the excess alkali metal halide remains as such. When the reaction is performed in the absence of an ancillary diluent, an excess amount of the alkali metal fluoride can serve to facilitate stirring or other agitation of the reaction mixture, and thus to this extent use of a suitable excess of alkali metal fluoride can be beneficial. Nevertheless, beyond a certain level of excess alkali metal fluoride, common sense and practicality come into play. Thus ordinarily the amount of alkali metal fluoride will not exceed about 10 or 15 moles per mole of replaceable halogen in the initial haloaromatic ingredient(s) used, and in most cases will be less than this. If on the other hand the amount of replaceable halogen in the haloaromatic ingredient(s) used exceeds the molar quantity of alkali metal fluoride used, the latter becomes the limiting reactant. Thus in most cases this factor will also be taken into consideration when selecting the proportions for use in any given reaction. Generally speaking, the reactants will often be employed in proportions falling in the range of about 0.8 to about 5 moles of alkali metal fluoride per mole of replaceable halogen in the haloaromatic ingredient(s) used therewith, and in some preferred cases such as where an ancillary diluent is employed, the reactants will be charged in proportions in the range of about 1 to about 3 moles of alkali metal fluoride per mole of replaceable halogen in the haloaromatic ingredient(s) used therewith.

### Organo Guanidinium Salt Catalyst Ingredient

An essential catalyst ingredient of this invention is at least one organo guanidinium salt catalyst ingredient. One or more other co-catalysts may also be included, if desired, as long as at least one organo guanidinium salt catalyst ingredient is charged, concurrently or in any sequence, into the reaction zone or reaction mixture. Use of the organo guanidinium salt catalyst without use of a co-catalyst is currently deemed preferable.

Monovalent guanidinium salt catalysts are preferred, particularly monovalent hexahydrocarbyl guanidinium salts; divalent guanidinium salt catalysts may be used, but are less preferred. Divalent hydrocarbyl guanidinium salts can be represented by the formula : 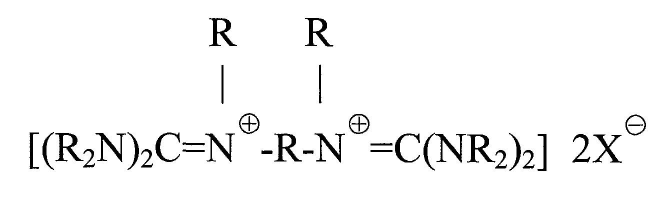 while monovalent hexahydrocarbyl guanidinium salts can be represented by the formula: 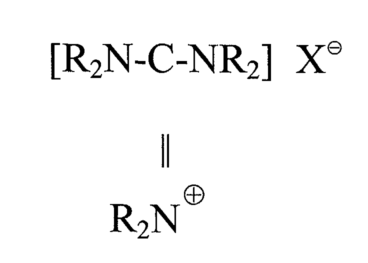 where, in both formulae, each R is, independently, a hydrocarbyl group, such as, for example, alkyl, cycloalkyl, aryl, alkaryl, aralkyl, cycloalkylalkyl, alkylcycloalkylalkyl, alkenyl, cycloalkenyl, alkylcycloalkenyl, alkylcycloalkenylalkyl, and any other hydrocarbyl group theoretically derivable by removal of a hydrogen atom from a hydrocarbon of any structure, each R preferably being an alkyl group, and X is a monovalent anion such as BF₄^{⊖}, alkoxide, aryloxide, cycloalkoxide, a halide atom, or any similar univalent anion, X preferably being a chlorine or bromine atom, and most preferably a bromine atom. Typically each R group contains, independently, up to about 24 carbon atoms, preferably up to about 10 carbon atoms, and most preferably up to about 6 carbon atoms. A few non-limiting examples of such organo guanidinium salt compounds are:
hexamethyl guanidinium fluoride
hexamethyl guanidinium chloride
hexamethyl guanidinium bromide
hexamethyl guanidinium iodide
hexamethyl guanidinium nitrate
hexamethyl guanidinium nitrite
hexamethyl guanidinium methoxide
hexamethyl guanidinium ethoxide
hexamethyl guanidinium 2-ethylhexoxide
hexamethyl guanidinium phenoxide
hexamethyl guanidinium hydrogen sulfate
hexamethyl guanidinium trifluoromethane sulfonate
hexamethyl guanidinium tetrafluoroborate
hexamethyl guanidinium hexafluorophosphate
hexamethyl guanidinium xylenesulfonate
hexaethyl guanidinium fluoride
hexaethyl guanidinium chloride
hexaethyl guanidinium bromide
hexaethyl guanidinium iodide
hexaethyl guanidinium nitrate
hexaethyl guanidinium nitrite
hexaethyl guanidinium methoxide
hexaethyl guanidinium ethoxide
hexaethyl guanidinium 2-ethylhexoxide
hexaethyl guanidinium phenoxide
hexaethyl guanidinium hydrogen sulfate
hexaethyl guanidinium trifluoromethane sulfonate
hexaethyl guanidinium tetrafluoroborate
hexaethyl guanidinium hexafluorophosphate
hexaethyl guanidinium xylenesulfonate
hexabutyl guanidinium fluoride
hexabutyl guanidinium chloride
hexabutyl guanidinium bromide
hexabutyl guanidinium iodide
hexabutyl guanidinium nitrate
hexabutyl guanidinium nitrite
hexabutyl guanidinium methoxide
hexabutyl guanidinium ethoxide
hexabutyl guanidinium 2-ethylhexoxide
hexabutyl guanidinium phenoxide
hexabutyl guanidinium hydrogen sulfate
hexabutyl guanidinium trifluoromethane sulfonate
hexabutyl guanidinium tetrafluoroborate
hexabutyl guanidinium hexafluorophosphate
hexabutyl guanidinium xylenesulfonate
hexaphenyl guanidinium fluoride
hexaphenyl guanidinium chloride
hexaphenyl guanidinium bromide
hexaphenyl guanidinium iodide
hexaphenyl guanidinium nitrate
hexaphenyl guanidinium nitrite
hexaphenyl guanidinium methoxide
hexaphenyl guanidinium ethoxide
hexaphenyl guanidinium 2-ethylhexoxide
hexaphenyl guanidinium phenoxide
hexaphenyl guanidinium hydrogen sulfate
hexaphenyl guanidinium trifluoromethane sulfonate
hexaphenyl guanidinium tetrafluoroborate
hexaphenyl guanidinium hexafluorophosphate
hexaphenyl guanidinium xylenesulfonate
hexatolyl guanidinium fluoride
hexatolyl guanidinium chloride
hexabenzyl guanidinium bromide
hexabenzyl guanidinium iodide
hexapentyl guanidinium nitrate
hexapentyl guanidinium nitrite
hexacyclohexyl guanidinium methoxide
hexacyclohexyl guanidinium ethoxide
hexatolyl guanidinium 2-ethylhexoxide
hexatolyl guanidinium phenoxide
hexabenzyl guanidinium hydrogen sulfate
hexabenzyl guanidinium trifluoromethane sulfonate
hexahexyl guanidinium tetrafluoroborate
hexacyclopentyl guanidinium hexafluorophosphate
hexanaphthyl guanidinium xylenesulfonate
hexapropyl guanidinium fluoride

1,1,3,3-tetramethyl-2,2-diethyl guanidinium fluoride
1,1,3,3-tetramethyl-2,2-dipropyl guanidinium chloride
1,1,3,3-tetramethyl-2,2-dihexyl guanidinium bromide
1,1,3,3-tetramethyl-2,2-diphenyl guanidinium iodide
1,1,3,3-tetraethyl-2,2-dimethyl guanidinium nitrate
1,1,3,3-tetraethyl-2,2-dibutyl guanidinium nitrite
1,1,3,3-tetraethyl-2,2-dicyclopentyl guanidinium methoxide
1,1,3,3-tetraethyl-2,2-diphenyl guanidinium ethoxide
1,1,3,3-tetraethyl-2,2-ditolyl guanidinium 2-ethylhexoxide
1,1,3,3-tetraisopropyl-2,2-dipentyl guanidinium phenoxide
1,1,3,3-tetrapropyl-2,2-diphenyl guanidinium hydrogen sulfate
1,1,3,3-tetrapropyl-2,2-dinaphthyl guanidinium trifluoromethane sulfonate
1,1,3,3-tetrabutyl-2,2-dimethyl guanidinium tetrafluoroborate
1,1,3,3-tetrabutyl-2,2-diphenyl guanidinium hexafluorophosphate
1,1,3,3-tetrapentyl-2,2-diethyl guanidinium xylenesulfonate
1,1,3,3-tetrapentyl-2,2-ditolyl guanidinium fluoride
1,1,3,3-tetracyclohexyl-2,2-dimethyl guanidinium chloride
1,1,3,3-tetracyclohexyl-2,2-diphenyl guanidinium bromide
1,1,3,3-tetraphenyl-2,2-dimethyl guanidinium iodide
1,1,3,3-tetraphenyl-2,2-diisopropyl guanidinium nitrate
1,1,3,3-tetraphenyl-2,2-ditolyl guanidinium nitrite
1,1,3,3-tetraphenyl-2,2-dinaphthyl guanidinium methoxide.

One preferred group of organo guanidinium salt catalyst in the form as charged to the reactor is comprised of the hexaalkyl guanidinium chlorides and/or bromides. Of these, the organo guanidinium salt catalyst ingredient is more preferably one or more hexaalkyl guanidinium bromides in which the alkyl groups can be the same or different and each has up to about 12 carbon atoms. In the hexaalkyl guanidinium halide, the alkyl groups are most preferably the same. At present, the most preferred compound is hexaethyl guanidinium bromide. Known routes to organo guanidinium salts are described in U.S. Pat. Nos. 4,271,190; 5,994,582; 6,093,848; 6,147,258 and Hullin *et al.*, *J. Chem. Soc.,* 1947, 394-396. Routes to hexaalkyl guanidinium salts are described in U.S. Pat. Nos. 5,082,968; 5,116,975; and 5,872,294.

The organo guanidinium salt catalyst is used in catalytically effective amounts, and such amounts typically fall in the range of about 2 to about 20 mole%, and preferably in the range of about 5 to about 10 mole%, based on the total amount (in moles) of the haloaromatic compound(s) with which the organo guanidinium salt catalyst is being associated in the reaction zone.

### Co-catalyst Ingredient

The organo guanidinium salt catalysts are effective when utilized as the only catalyst component charged directly or indirectly (i.e., after admixture with one or more other components being charged to the reaction system). Such catalytic mode of operation is preferred. However, as noted above, one or more co-catalyst ingredients may be used, if desired.

One type of such co-catalyst materials is comprised of one or more crown ethers or crypt compounds. These compounds, sometimes referred to as "cage compounds," can prove helpful in further enhancing the reactivity of the alkali metal fluoride. See in this connection, U.S. Pat. No. 4,174,349 to Evans, et al. A full description of the crown ethers and the crypt compounds is provided in the Evans, et al. patent and references cited therein relating to these materials, namely U.S. Pat. No. 3,687,978; J. J. Christensen, et al., *Chem. Rev.,* 1974, 74, 351; J. S. Bradshaw, et al., *Heterocycl. Chem.,* 1974, 11, 649; C. J. Pedersen, et al., *Angew. Chem. Int. Ed. Engl.,* 1972, 11, 16; the Technical Bulletin of PCR Incorporated entitled KRYPTOFIX; and *J. Org. Chem.,* 1977, Vol 42, No. 10, 2A. The crown ether or crypt compound is used in a catalytically effective amount, which typically is in the range of 0.01 to 1 mol per mol of haloaromatic compound(s) in the reaction mixture.

Another type of co-catalyst that can be used is composed of (i) at least one polyvalent inorganic fluoride of boron, aluminum, tin, phosphorus, titanium, zirconium, hafnium, or silicon, or (ii) at least one double salt of the polyvalent inorganic fluoride and alkali metal fluoride, or (iii) a combination of (i) and (ii), with the proviso that the inorganic fluoride of (i), (ii) and (iii) is in a stable valency state so that (i), (ii) and (iii), as the case may be, has no oxidizing properties. U.S. Pat. No. 3,453,337 to Bennett, et al., reports that in the uncatalyzed reaction between hexachlorobenzene and KF or NaF, the inclusion of compounds of the types (i), (ii) and (iii) above provides enhanced product yields using milder reaction conditions and shorter reaction times. Examples of suitable polyvalent compounds include LiBF₄, NaBF₄, KBF₄, K₂SnF₆, KPF₆, K₂SiF₆, Na₂TiF₆, K₂TiF₆, Na₂ZrF₆, K₂ZrF₆, Na₂HfF₆, K₂HfF₆, among others. Such compounds can be used in catalytically effective amounts of up to 50% or more of the weight of the alkali metal fluoride charged to the reaction mixture. Typically the amount will fall in the range of about 2 to about 25% of the weight of alkali metal fluoride used.

Other co-catalysts which may be considered for use include quaternary ammonium salts such as described for example by J. Dockx, *Synthesis,* **1973,** 441; C.M. Starks and C. Liotta, Phase Transfer Catalysts, **1978,** Academic Press, New York; and W.P. Weber and G.W. Gokel, *Phase Transfer Catalysis in Organic Synthesis,* **1977,** Springer-Verlag, Berlin-Heidelberg-New York); and metal carbonyls such as described by M.F. Semmelhack and H.T. Hall, *J. Am. Chem. Soc.,* **1974,** 96, 7091.

The organo guanidinium salt catalyst and the above co-catalyst(s), if used, can vary both in function and in composition. As to function, they can serve to promote or enhance the fluorination exchange reaction, e.g., (a) by increasing reaction rate without affecting yield or selectivity, (b) by increasing yield or selectivity, or both, without affecting reaction rate, or (c) by increasing reaction rate and improving yield or selectivity, or both. Thus the term "catalyst" or "co-catalyst" is used herein to denote that the material in the manner used improves or enhances the reaction process in some way or other so that the inclusion or presence of that material or its progeny in the reaction mixture provides at least one beneficial consequence of its use. The mechanism by which it exerts its effect(s) is of no consequence, provided of course that the advantage(s) of its use outweigh(s) the disadvantage(s), if any, of its use.

As regards catalyst and co-catalyst composition, the material is identified herein as to its composition prior to being combined with any other substance being used in the process. After addition to, and/or mixing with, one or more other ingredients used in the process and/or during the course of the process itself, the catalyst may change in its composition, and if so, the resultant changed material, whatever its makeup and however many changes it may undergo, may be responsible in whole or in part for the functioning of the catalyst.

### Process Conditions

The process can be conducted by dry mixing the finely-divided essentially anhydrous alkali metal fluoride, the haloaromatic compound having at least one halogen atom of atomic number greater than 9 on an aromatic ring, and an organo guanidinium salt catalyst, and heating the mixture at one or more reaction temperatures at which at least one such halogen atom of the haloaromatic compound is replaced by a fluorine atom. Alternatively, the foregoing ingredients may be heated to one or more such reaction temperatures while in admixture with an ancillary solvent/diluent. The solvent or diluent used is preferably a polar aprotic solvent such as, for example, sulfolane (tetramethylene sulfone), N,N-dimethylformamide, N,N-dimethylacetamide, dimethylsulfone, dimethylsulfoxide, triglyme (triethylene glycol dimethyl ether), 1,3-dimethyl-2-imidazolidinone, N-methyl pyrrolidinone, or benzonitrile, or mixtures of two or more of such materials. Other solvent/diluents for use in the process are haloaromatics that are in the liquid state at least at, and preferably below, the reaction temperature(s) being employed. Examples include hexafluorobenzene, octafluorotoluene, perfluorodecalin, dichlorotetrafluorobenzene, trichlorotrifluorobenzene and tetrachlorodifluorobenzene. The last three such compounds are especially desirable as solvent/diluents when producing pentachlorofluorobenzene as they not only serve as solvent/diluents, but as reactants as well. For optimum reaction performance the halogen exchange reaction should be run under essentially anhydrous conditions , that is, the overall moisture content of the reaction mix should preferably be less than 1%, most preferably less than 0.1%.

Whether the reaction mixture is formed with or without a solvent/diluent, the reaction mixture should be thoroughly agitated during the course of the reaction to ensure intimate contact among the different materials in the mixture. Thus use of mechanical agitation equipment such as mechanical stirrers, rocking autoclaves, or similar apparatus is highly recommended.

Reaction temperatures will typically be in the range of about 150°C to about 350°C and preferably in the range of about 170°C to about 250°C, and more preferably in the range of about 170°C to about 220°C. The reaction may be conducted at atmospheric, sub-atmospheric or super-atmospheric pressures. In many cases it is desirable as well as convenient to carry out the reaction in a closed system at autogenous pressures. Alternatively, the reaction may be carried out in a liquid phase under reflux conditions. Reaction periods will typically fall in the range of about 2 to about 48 hours, and preferably in the range of about 5 to about 20 hours. It will be appreciated that on the basis of this disclosure, departures from any of the ranges of proportions and/or reaction conditions given above may be made whenever such departures are deemed necessary or desirable.

### Synthesis of organo guanidinium salt

As mentioned above, another embodiment of this invention is a novel process for producing a monovalent hexahydrocarbyl guanidinium salt from a salt of unsubstituted guanidine. A particular advantage of this reaction is that six hydrocarbyl groups can be added in a simple, one-step process. The process comprises mixing together a halohydrocarbon, an inorganic base, and a salt of unsubstituted guanidine. The absence of water during the process is not required, and the use of an organic solvent is optional.

Divalent guanidinium salts, also called bis(guanidinium) salts, can be synthesized in a manner similar to that of the monovalent guanidinium salts. An advantage of this method for making divalent guanidinium salts is that the process can be carried out in one vessel. The process for synthesizing divalent guanidinium salts comprises mixing together a first halohydrocarbon, an inorganic base, and a salt of unsubstituted guanidine, followed by mixing of a second halohydrocarbon with the mixture so formed; this is followed by the mixing of a third halohydrocarbon with the mixture formed from the mixing of the second halohydrocarbon. As in the synthesis of the monovalent salts, the absence of water during the process is not required, and the use of an organic solvent is optional.

The halohydrocarbon may be a chlorohydrocarbon, a bromohydrocarbon, or an iodohydrocarbon; bromohydrocarbons are preferred halohydrocarbons. The halohydrocarbon may contain from 1 to preferably about 24 carbon atoms, and may be a saturated, unsaturated, or aromatic halohydrocarbon. Monohalohydrocarbons are preferred for the synthesis of monovalent guanidinium salts, and as the first and third halohydrocarbon in the synthesis of divalent guanidinium salts. Dihalohydrocarbons may be used, but are not preferred in the synthesis of monovalent salts, or as the first or third halohydrocarbon for the divalent salts. Examples of suitable monohalohydrocarbons include bromoethane, iodoethane, 1-bromopropane, 2-iodopropane, 2-bromobutane, 1-bromo-2-ethylbutane, 3-bromopentane, cyclopentyl bromide, cyclohexyl bromide, 1-iodohexane, chlorobenzene, bromonaphthalene, 4-iodotoluene, and the like. Saturated bromohydrocarbons are preferred, particularly those containing from about 1 to about 12 carbon atoms. The most highly preferred monohalohydrocarbon is bromoethane. Dihalohydrocarbons are necessary as the second halohydrocarbon in the synthesis of bis(guanidinium) salts. Suitable dihalohydrocarbons include, but are not limited to, 1,2-dichloroethane, 1,2-dibromoethane, 1-chloro-2-iodopropane, 1,4-dibromobutane, 1,3-dichloro-2-ethylbutane, 2,4-diiodopentane, 1,3-dibromocyclopentane, 1,4-dichlorocyclohexane, 1,6-diiodohexane, 1-bromo-3-chlorobenzene, 2-bromo-4-iodonaphthalene, 3,4-dichlorotoluene, and the like. The most preferred dihalohydrocarbon is 1,2-dibromoethane. Halohydrocarbons containing functional groups which do not interfere with the synthesis of the guanidinium salt may be used. It is preferred to use halohydrocarbons which do not contain other functional groups, *i.e.,* the hydrocarbon portion of the halohydrocarbon is comprised of only carbon and hydrogen. Mixtures of halohydrocarbons where the hydrocarbyl groups are different can be used, if desired, but are not preferred.

For the synthesis of monovalent guanidinium salts and for the synthesis of divalent guanidinium salts, various inorganic bases may be used. Such inorganic bases include oxides, hydroxides, carbonates, and bicarbonates of the alkali metals, alkaline earth metals, zinc, and the like. Examples of suitable bases are sodium oxide, potassium oxide, magnesium oxide, calcium oxide, zinc oxide, lithium hydroxide, sodium hydroxide, potassium hydroxide, rubidium hydroxide, magnesium hydroxide, calcium hydroxide, barium hydroxide, zinc hydroxide, lithium bicarbonate, sodium carbonate, potassium bicarbonate, rubidium carbonate, magnesium carbonate, calcium carbonate, and zinc carbonate. Alkali metal bases are preferred; more preferred are alkali metal hydroxides, and sodium hydroxide is most preferred as the inorganic base. Mixtures of inorganic bases can be used. The inorganic base may be introduced to the reaction vessel in solid form or as a solution; an aqueous solution of the inorganic base is particularly preferred. When an aqueous solution of inorganic base is used, the concentration of inorganic base in the aqueous solution is preferably in the range of about 5 to about 15 moles per liter, and more preferably in the range of about 8 to about 13 moles per liter.

The salt of unsubstituted guanidine, used in both the process for making monovalent guanidinium salts and the process for making divalent guanidinium salts, is also commonly called a guanidine salt. The salt of unsubstituted guanidine is a complex of unsubstituted guanidine and an acid, *e.g.*, guanidine nitrate is H₂NC(=NH)NH₂•HNO₃. Salts of unsubstituted guanidine that are suitable for use in this process include, but are not limited to, guanidine acetate, guanidine carbonate, guanidine hydrobromide, guanidine hydrochloride, guanidine nitrate, guanidine picrate, guanidine thiocyanate, guanidine isothiocyanate, guanidine sulfate, guanidine stearate, and guanidine sulfamate. Preferred salts of unsubstituted guanidine are guanidine hydrobromide and guanidine hydrochloride; most preferred is guanidine hydrochloride. Mixtures of two or more guanidine salts may be used, and are not expected to affect the outcome of the reaction.

When synthesizing a monovalent guanidinium salt, the mole ratio of halohydrocarbon to salt of unsubstituted guanidine is in the range of about 6:1 to about 10:1. The use of a molar excess of halohydrocarbon relative to the salt of unsubstituted guanidine is preferred.

For the synthesis of a bis(guanidinium) salt, the mole ratio of first halohydrocarbon to salt of unsubstituted guanidine is in the range of about 4:1 to about 7:1, as it is expected that the guanidine will become tetrasubstituted. The mole ratio of dihalohydrocarbon to guanidine is in the range of about 0.5:1 to about 1:1; one dihalohydrocarbon molecule is needed for every two molecules of guanidine salt. For the third halohydrocarbon, the mole ratio of halohydrocarbon to salt of unsubstituted guanidine is in the range of about 1:1 to about 4:1; two halohydrocarbons groups are expected to react with the guanidine moiety. Use of a molar excess of halohydrocarbon relative to the salt of unsubstituted guanidine is normally preferred.

The amount of inorganic base needed for reaction with the unsubstituted salt of guanidine is at least about six moles of proton-accepting ability per about one mole of guanidine salt. As an illustration of the term "proton-accepting ability," one mole of hydroxide ion has one mole of proton-accepting ability, while one mole of carbonate ion has two moles of proton-accepting ability. One mole of proton-accepting ability is needed to transform the guanidine salt into free guanidine if it is a salt of a monoprotic acid, while two (or more) moles of proton-accepting ability are needed to transform the guanidine salt into free guanidine if it is a salt of a di- (or more) protic acid. Five additional moles of basicity are presumably consumed by hydrohalic acid produced during the reaction of the halohydrocarbon with the guanidine salt. The mole ratio of proton-accepting ability to salt of unsubstituted guanidine is normally at least about 7:1, and more preferably in the range of about 7:1 to about 10:1. The use of excess of proton-accepting ability (and thus excess of inorganic base) is preferred. For example, the use of at least about 3.5 moles of sodium carbonate per mole of guanidine salt is preferred, while at least about seven moles of potassium hydroxide per mole of guanidine salt are preferable. The molar amounts of inorganic base required do not vary for synthesis of the divalent salt because the same amount of acid salt (of guanidine) and hydrohalic acid are present as in the synthesis of the monovalent salt.

In the synthesis of bis(guanidinium) salts, the same overall amounts of inorganic base are used as described above. Enough inorganic base to provide at least five moles of proton-accepting ability per mole of guanidine salt are needed before the second halohydrocarbon is made a part of the mixture. Before the third halohydrocarbon is made a part of the mixture, enough additional inorganic base to provide at least one mole of proton-accepting ability per mole of guanidine salt is present (such that the total amount of proton-accepting ability is at least six moles per mole of guanidine salt). Additional inorganic base may be used at any point in the process, and the use of excess base is highly preferred.

For the optional organic solvent in the process for making monovalent guanidinium salts and the process for making divalent guanidinium salts, a variety of solvents may be used. Suitable solvents for the liquid organic medium include hydrocarbons and water. Suitable hydrocarbons include pentane, hexane, heptane, benzene, toluene, and xylenes. The halohydrocarbon that is to be reacted with the guanidine salt may also be used as the solvent. Currently, it is deemed preferable to conduct the reaction without an organic solvent.

For the synthesis of the monovalent guanidinium salt, it is preferred that the halohydrocarbon and the salt of unsubstituted guanidine are present in the reaction vessel prior to the addition of the inorganic base. The inorganic base is slowly introduced to the reaction vessel; the addition usually takes about thirty minutes to about three hours on the laboratory scale. The solution is heated, preferably to a temperature in the range of about 30°C to about 50°C. Preferably, the halohydrocarbon, the guanidine salt, and the inorganic base are added to the reaction vessel before heating is begun. Reaction times on the laboratory scale are typically in the range of about 12 to about 72 hours.

When synthesizing the divalent guanidinium salt, it is preferred that the first halohydrocarbon and the salt of unsubstituted guanidine are present in the reaction vessel prior to the addition of inorganic base. Inorganic base is slowly introduced to the reaction vessel; the addition usually takes about thirty minutes to about three hours on the laboratory scale. The mixture is heated, preferably to a temperature in the range of about 30°C to about 50°C. Preferably, the first halohydrocarbon, the guanidine salt, and inorganic base are added to the reaction vessel before heating is begun. After the mixture has attained the desired temperature, the second halohydrocarbon is mixed with the heated mixture; it is preferred to add the second halohydrocarbon to the mixture. More inorganic base may be mixed with this mixture, either before, after, or concurrently with the mixing of the second halohydrocarbon. The third halohydrocarbon is mixed with the mixture formed from the mixing of the second halohydrocarbon; again, it is preferable to add the third hydrocarbon to this mixture. Reaction times on the laboratory scale are typically in the range of about 12 to about 72 hours.

The following examples are for the purpose of illustration and not limitation.

### EXAMPLE 1

### Synthesis of hexaethyl guanidinium bromide

To a 1000ml glass reactor fitted with agitator, reflux condenser, dropping funnel, and thermometer, bromoethane (440g, 4.036 moles) and guanidinium hydrochloride (48.7g, 0.51 moles) were charged. Agitation was commenced, and sodium hydroxide (50%. 280g, 3.5 moles) was charged to the reactor during one hour. The reaction mixture was heated to reflux and refluxed for 48 hours. The reaction mixture was then cooled to ambient temperature and agitation was ceased. The phases were allowed to separate, and the upper organic phase was extracted. The organic phase was placed on a rotary evaporator to remove excess bromoethane (at ca. 30-35°C; slight vacuum). The remaining oily product was added to diethyl ether (ca. 500ml), whereupon crystallization occurred. The crystalline product was filtered and dried in a vacuum oven at 50°C over P₂O₅. The yield of hexaethyl guanidinium bromide was 44.5% (70g, 0.2273 moles), based on guanidinium hydrochloride.

### EXAMPLES 2-10

### Halogen exchange reactions using a hexaalkyl guanidinium halide catalyst

**Apparatus.** The halogen exchange reactions were performed in a 220ml stainless steel reactor fitted with an agitator, a thermocouple, a pressure gauge, and a sampling port. Heating was by means of an electric jacket.

**Reagents**. The potassium fluoride was calcined for 2 hours at 200°C prior to use. Nitrobenzene (98%) was distilled prior to use.

**General Procedures**. For each run, the materials for that run, in the amounts specified in Table 2, were charged to the reactor. The catalyst for all reactions was hexaethyl guanidinium bromide. The reactor head was then placed on the reactor body and secured; the reactor assembly was then placed in the heating jacket and the agitator was connected to the motor. Agitation was commenced. The agitator speed was set at 200±20 rpm. The reactor contents were heated for approximately 2 hours to reach the reaction temperature. The temperature was maintained at the desired value ±2.5°C. During the reaction, the reactor contents were sampled periodically and analyzed. The samples were removed via the sample port. Upon completion of the reaction, the reactor contents were cooled to ambient temperature and the reactor was disassembled. The reactor contents were then poured into a 500ml glass reactor, washing the stainless steel reactor with water to ensure complete removal of products. Water was added to dissolve KF and KCl. Azeotropic distillation of volatile organic substances was carried out. The residue of the distillation consisted of polymeric by-product dissolved in an organic layer and an aqueous layer; these layers were separated. The organic layer was returned to the flask, and distillation to dryness was performed. The weight of the polymeric by-product was determined by the difference in flask weight before addition of the reactor contents and after the distillation to dryness.

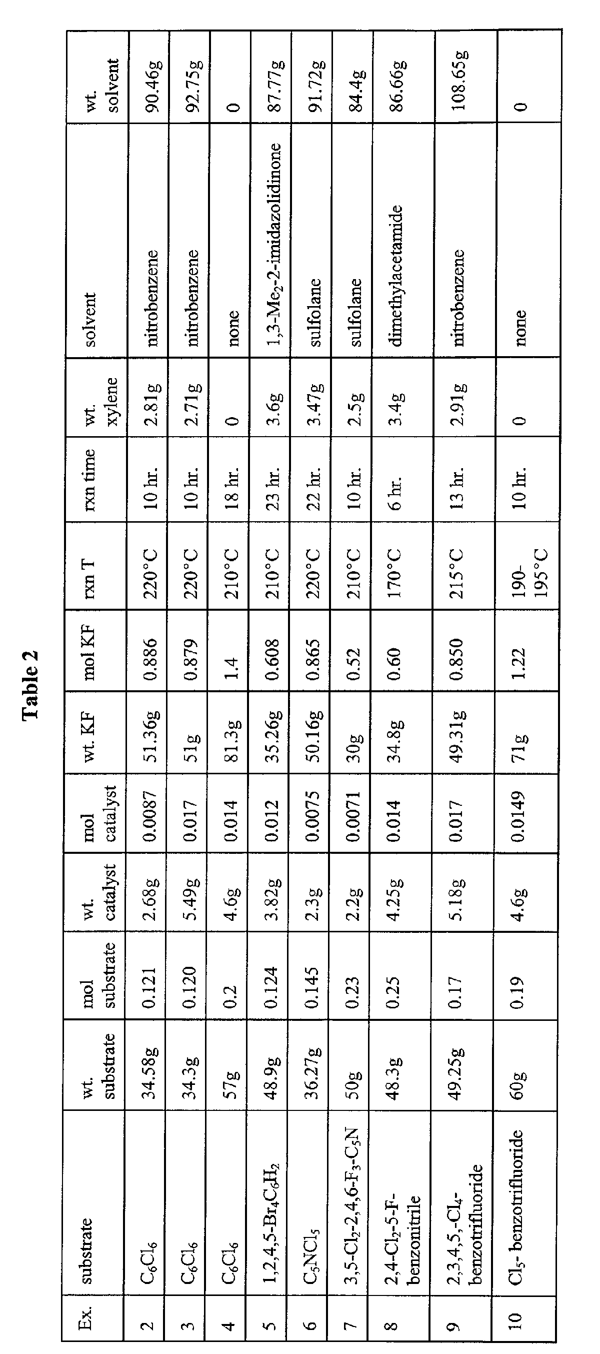

**Analyses**. Analysis of reaction products for haloaromatic compounds was by means of gas chromatography (GC) using a Hewlett Packard 5890 gas chromatograph. Prior to analysis, each sample was diluted in enough dichloromethane to produce a 10% (by volume) solution of the sample. For the reactions of pentachloropyridine and dichlorotrifluoropyridine, the column used was a VS-225 (OV-225) column. The column used for all other halogen exchange reactions was a VS-101 (OV-101) column. The GC conditions used for all halogen exchange reaction analyses are in Table 3.

**Table 3**

| | |
|---|---|
| Column | 30m x 0.53mm x 2.2µm |
| Carrier Gas | Helium, flow rate: 3.5ml/min |
| Injector Temperature | 200°C |
| Detector Temperature | 280°C (flame ionization detector) |
| Oven Temperature | Initial: 30°C Final: 200°C |
| Heating Program | 30°C for 2 min. Heat to 200°C at 15°C/min 200°C for 5 minutes |

The results of Examples 2-10 are summarized in Tables 4-9. Xylene was included in all reactions to act as an internal standard for the GC analyses. The %C-Cl conversion was calculated from GC analysis of the reaction mixture and relates to the molar conversion of C-Cl bonds to C-F bonds. Fluorination selectivity was calculated from the molar yield of fluorination products isolated from the reaction. Product removed is the weight of fluorinated products isolated from the reaction mixture. The percent yield reported for Examples 4 and 10 is of the fluoroaromatic products, and is based on the amount of chloroaromatic starting material.

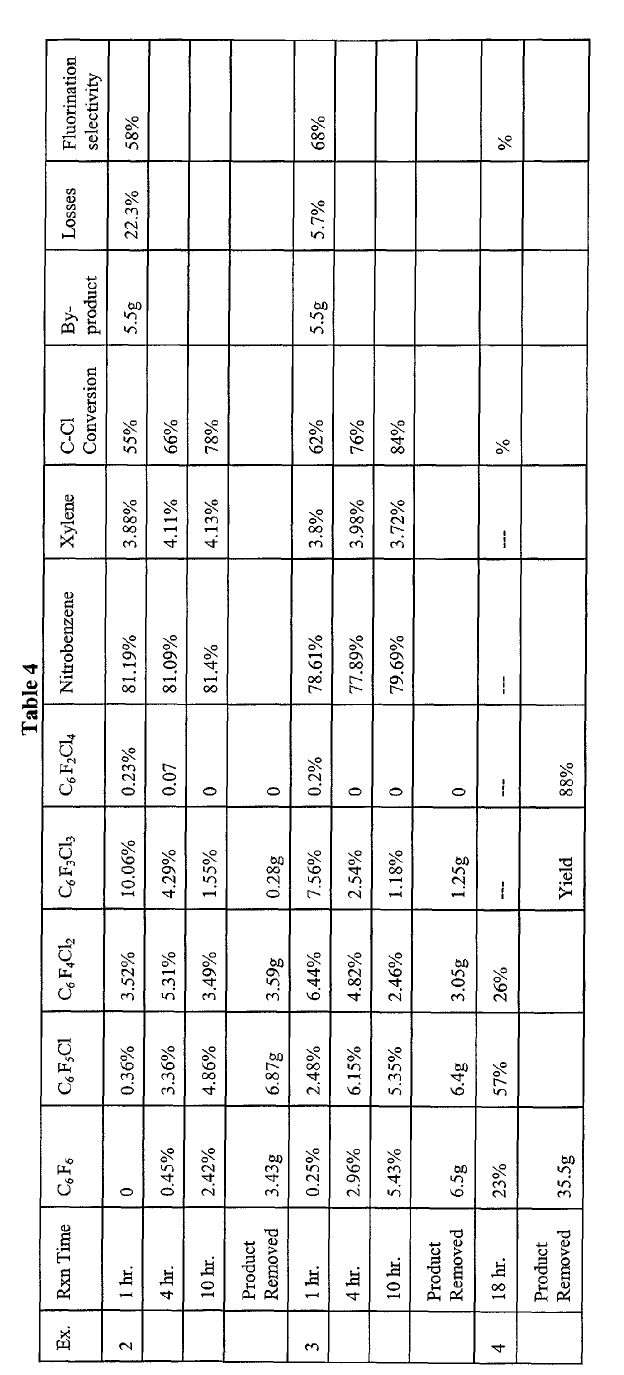

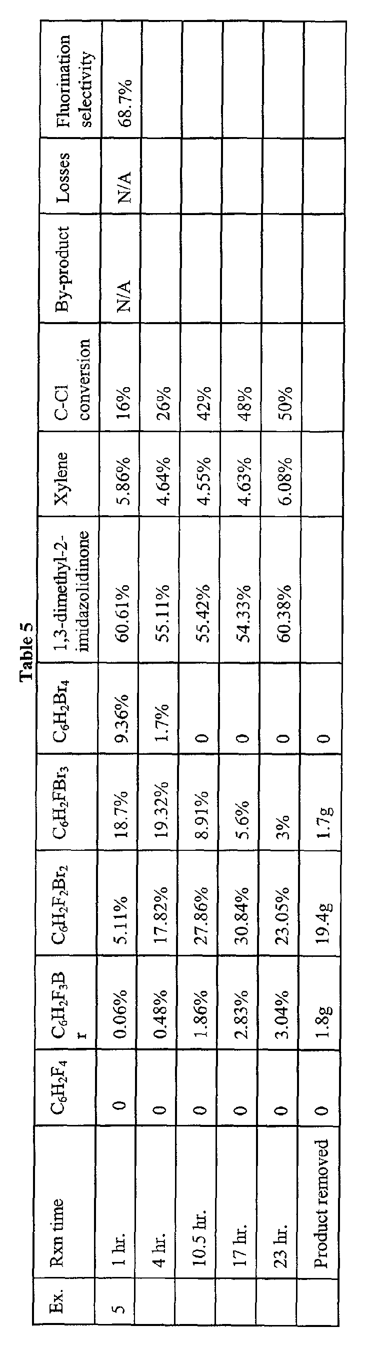

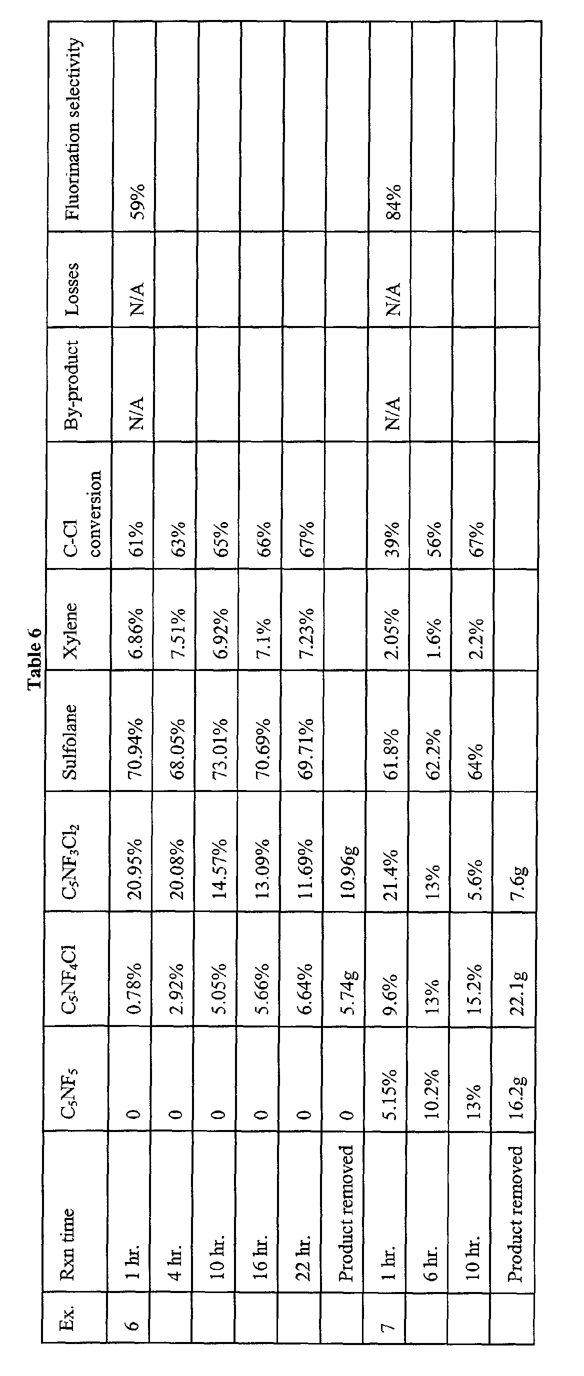

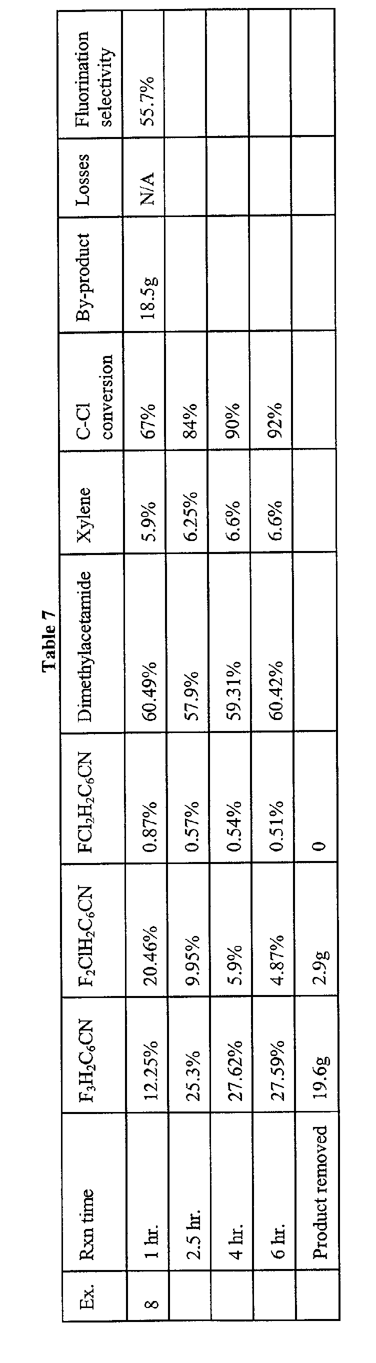

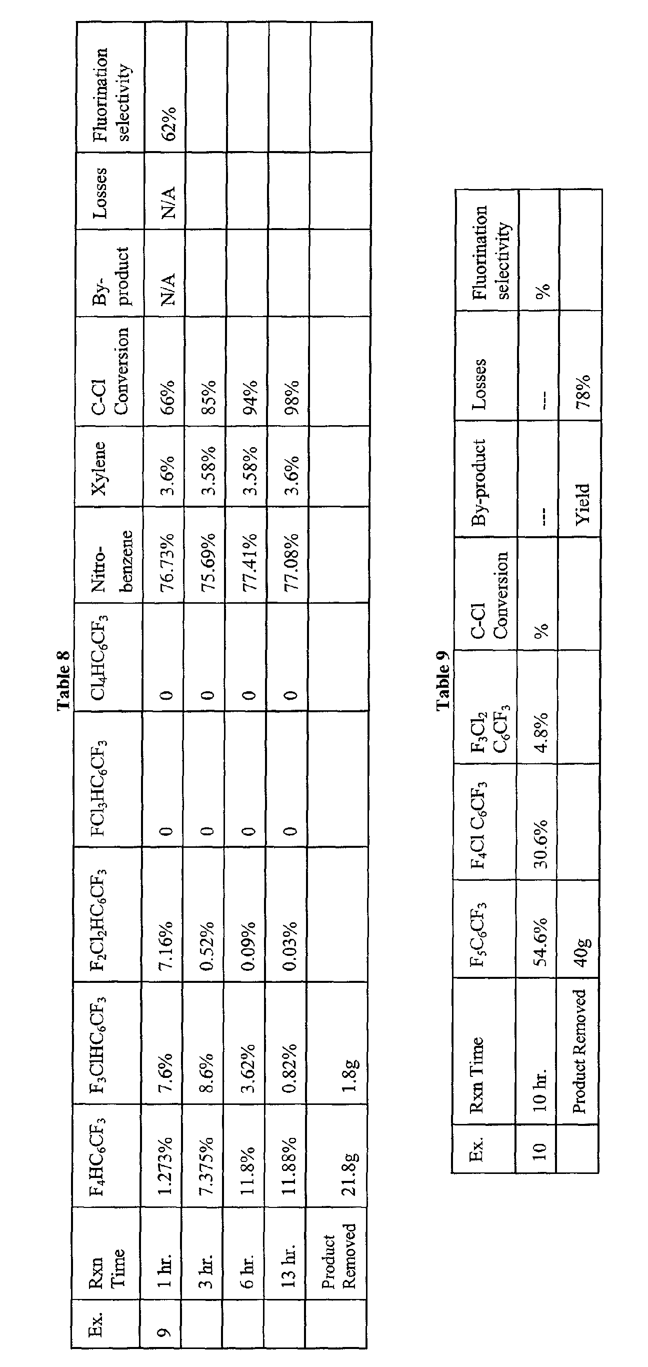

It is to be noted that in the above examples, the catalyzed process of this invention was conducted at a maximum temperature of 210°C without use of any added ancillary solvent or diluent. A conventional non-catalyzed non-solvent reaction of hexachlorobenzene with potassium fluoride typically involves use of 20-liter autoclaves operating at a temperature of 450°C and a pressure of up to 1,500 psi and employs an 85% excess of potassium fluoride. Based on total raw material input, batch yield of desired products is around 12%.

### COMPARATIVE EXAMPLES 11-18

### Halogen exchange reactions using a tetrakis(dialkylamino)phosphonium halide catalyst

The apparatus, reagents, general procedures, and analyses were as described for Examples 2-10, except that the catalyst for all runs was tetrakis(diethylamino)phosphonium bromide. For each run, the materials for that run, in the amounts specified in Table 10, were charged to the reactor.

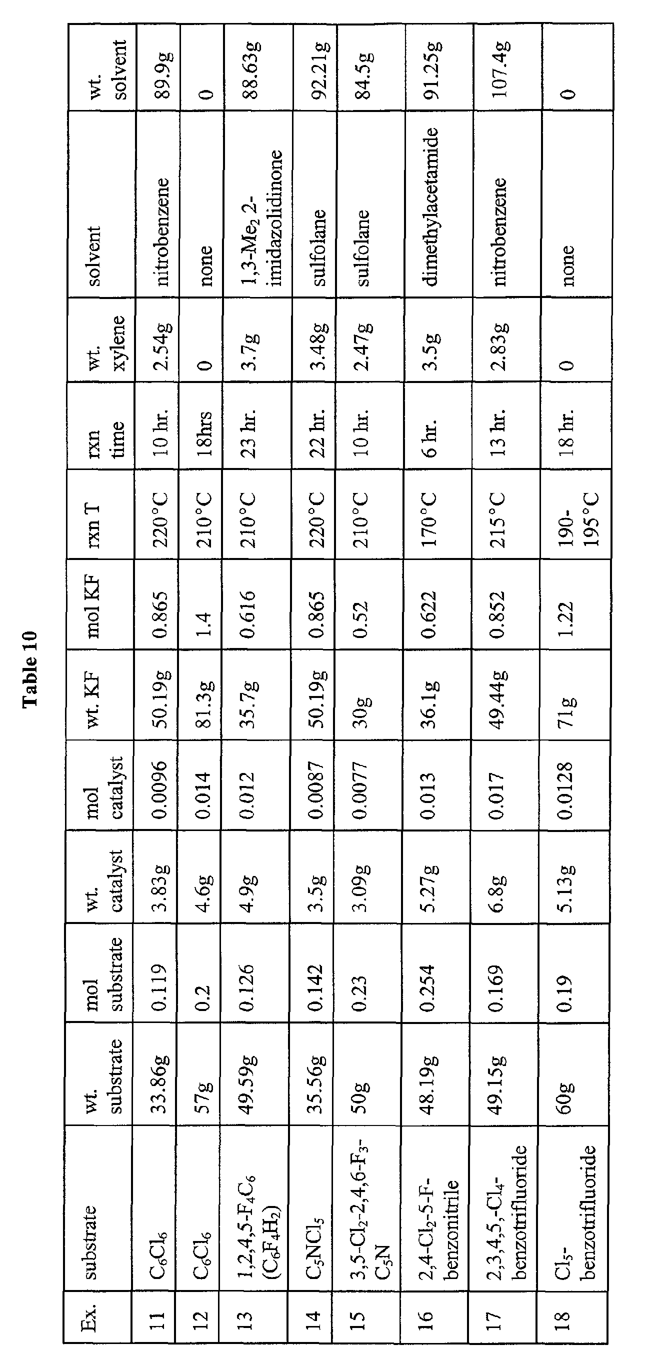

The results of the Comparative Examples are summarized in Tables 11-16. The %C-Cl conversion and fluorination selectivity are calculated as described above for the results of Examples 2-10. The weight of product removed is also as described for Examples 2-10. For Examples 12 and 18, the product removed is the weight of fluorinated products isolated from the reaction mixture. The percent yield reported for Examples 12 and 18 is of the fluoroaromatic products, and is based on the amount of chloroaromatic starting material.

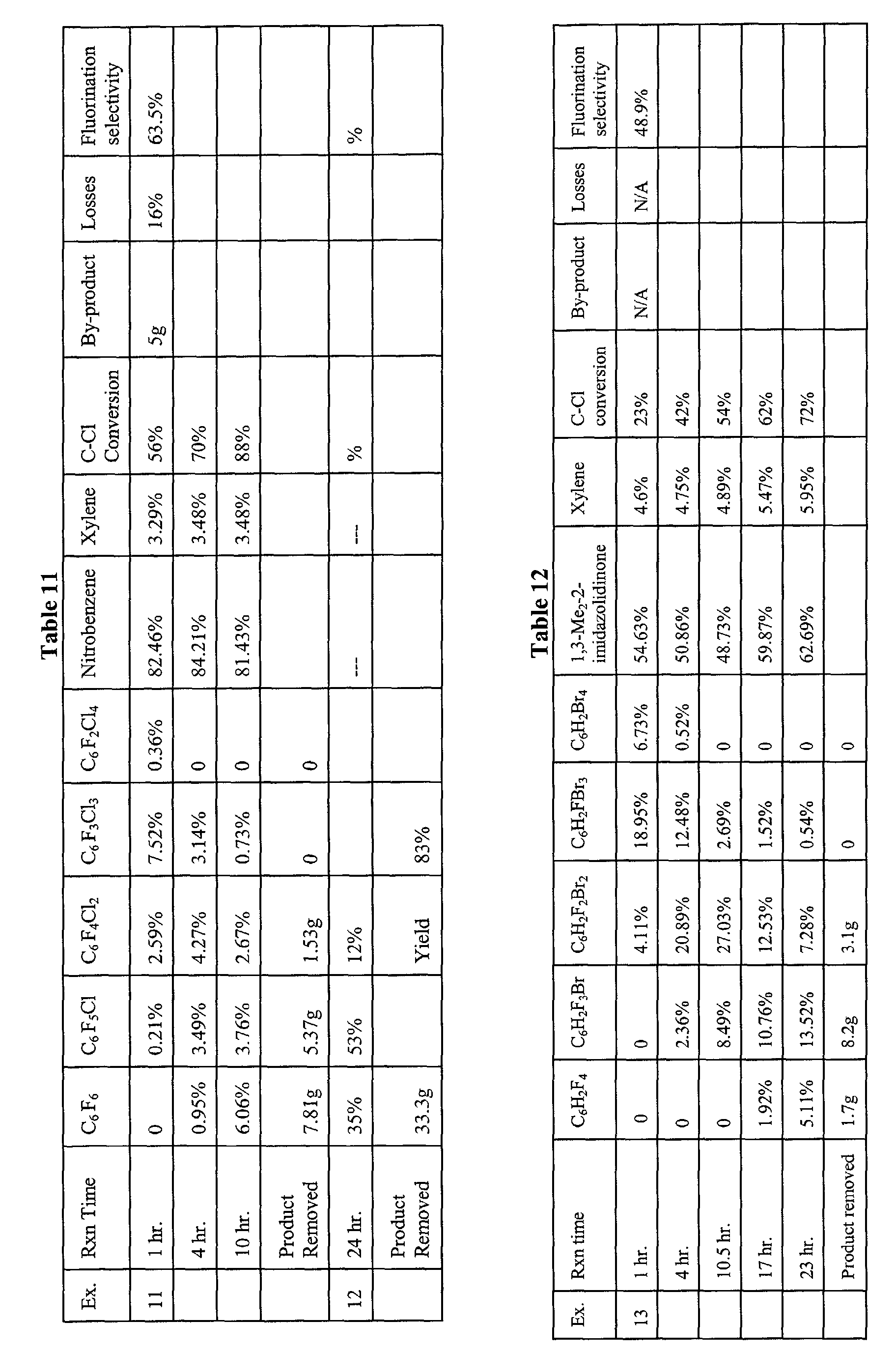

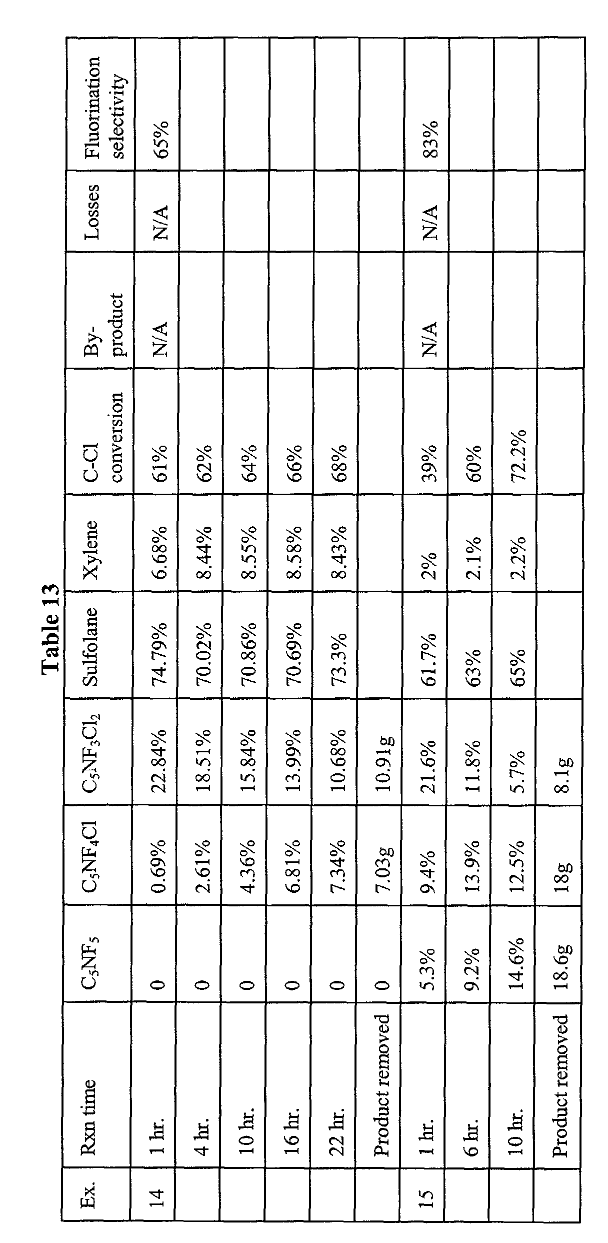

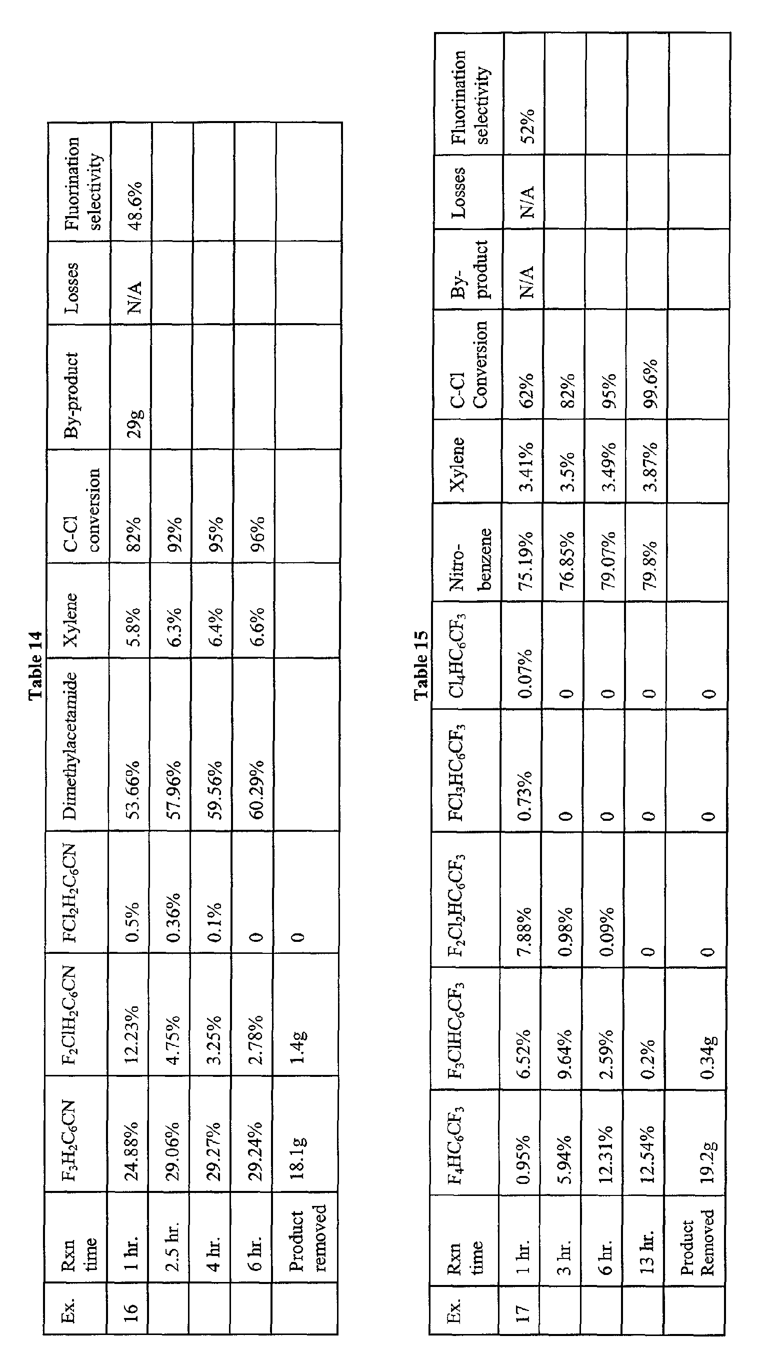

**Table 16**

| Ex. | Rxn time | F₅C₆CF₃ | F₄Cl C₆CF₃ | C-Cl Conversion | By-product | Losses | Fluorination selectivity |
|---|---|---|---|---|---|---|---|
| 18 | 18 hr. | 89.4% | 4.5% | % | --- | --- | % |
| | Product removed | 29.5g | | Yield | 61.6% | | |

As indicated above, one highly preferred form of alkali metal fluoride is an activated KF formed by recrystallizing KF from methanol solution by slow evaporation of the solvent at reduced pressure followed by drying at an elevated temperature. The following is an illustrative laboratory procedure that may be used for conducting this operation, which procedure is based on that described by T. P. Smyth, A. Carey and B. K. Hodnett in *Tetrahedron,* Volume 51, No. 22, pp. 6363-6376 (1995). KF is dissolved in excess dry methanol (e.g., in proportions of about 1 part by weight of KF per about 13 to about 20 parts by weight of methanol). This operation is preferably conducted under a dry nitrogen atmosphere, and preferably the methanol used is 99.9+ % A.C.S. HPLC grade packaged under nitrogen. The methanol is slowly removed at reduced pressure at a temperature in the range of about 25 to about 35°C (e.g., 30°C) and then dried *in vacuo* at one or more temperatures in the range of about 70 to about 120°C (e.g., 100°C) for a period of at least about 5 hours (e.g. 6 hours). Preferably the activated KF is used promptly after it has been prepared.

A number of modifications in the process are possible without departing from the scope of this invention. By way of illustration and not limitation, the following modifications are presented:
a) The catalyst or catalyst residues may be recycled.
b) When the desired product is a polyfluoroaromatic compound, intermediates formed that contain a lesser than desired number of fluorine atoms per molecule may be recycled.
c) If the desired product has suitably high volatility, it may be removed from the reaction zone during the course of the reaction, e.g., essentially as soon as it is formed, so as to prevent or at least minimize overfluorination.
d) Special procedures, e.g., drying by azeotropic distillation or by high temperature spray drying, may be employed for drying the alkali metal fluoride before use.
e) Multistage drying procedures may be used for drying the alkali metal fluoride before use.
f) The alkali metal fluoride may be micronized or reduced to a colloidal state in one or more stages prior to use.
g) Combinations of one or more drying stages with one or more micronizing stages, or vice versa, may be applied to the alkali metal fluoride before use.
h) Whether operating with or without an ancillary solvent, the alkali metal fluoride may be an optimized mixture composed of a major amount of dry, finely-divided potassium fluoride with a minor reaction-enhancing amount of dry, finely-divided cesium fluoride.
i) When producing a desired product having one or more intermediates that are in the liquid state at or below the selected reaction temperature(s), such intermediates may be employed as solvent/diluents in the process.
j) The proportions of the selected ingredients for use in any given situation may, and should be, optimized by performing carefully designed pilot experiments and scale-up trials before settling upon the mode of operation in a large scale commercial facility.
k) In lieu of or in addition to one or more simple alkali metal fluorides, e.g. KF, the alkali metal reactant may be or include a more complex alkali metal salt such as a double salt, examples of which include KBF₄, CsBF₄, NaBF₄, K₃AlF₆, K₂SnF₆, Cs₂SnF₆, KPF₆, CsPF₆, K₂SiF₆, Cs₂SiF₆, Na₂TiF₆, K₂TiF₆, Na₂ZrF₆, K₂ZrF₆, Na₂HfF_{6,} K₂HfF₆, among others.
l) In addition to the organo guanidinium salt, tetrakis(dialkylamino)phosphonium halides, especially tetrakis(diethylamino)phosphonium chloride and tetrakis(diethylamino)phosphonium bromide, may be used as co-catalyst ingredients.

An example of one such modification which constitutes an embodiment of this invention relates to the synthesis of chloropentafluorobenzene and/or hexafluorobenzene from hexachlorobenzene. In this process the alkali metal fluoride ingredient used preferably comprises potassium fluoride, and the organo guanidinium salt catalyst ingredient used is preferably at least one hexaalkylguanidinium halide (especially hexaethyl guanidinium bromide), and the agitated mixture formed from hexachlorobenzene, potassium fluoride, and the organo guanidinium salt catalyst ingredient is heated at one or more reaction temperatures in the range of 170 to 220°C for at least a substantial portion of the reaction. In this particular embodiment the agitated mixture comprises solids suspended or dispersed in continuous liquid phase, which preferably comprises a major amount (preferably 60 volume % or more at the outset of the reaction) of at least one chloro-fluoro-perhalobenzene that is in the liquid state at least while the agitated mixture is at one or more reaction temperatures in the range of 170 to 220°C. Examples of such chloro-fluoro-perhalobenzenes include dichlorotetrafluorobenzene (b.p. at atmospheric pressure, approximately 151°C), trichlorotrifluorobenzene (m.p., approximately 62°C), and tetrachlorodifluorobenzene (m.p., approximately 138°C). Of these, dichlorotetrafluorobenzene is particularly desirable as it is a liquid at room temperature and can readily be kept in the liquid state at temperatures in the range of 170 to 220°C by conducting the reaction at suitable superatmospheric pressures.

It is to be understood that the ingredients referred to by chemical name or formula anywhere in the specification or claims hereof, whether referred to in the singular or plural, are identified as they exist prior to coming into contact with another substance referred to by chemical name or chemical type (e.g., another reactant, a solvent, a diluent, or etc.). It matters not what preliminary chemical changes, transformations and/or reactions, if any, take place in the resulting mixture or solution or reaction medium as such changes, transformations and/or reactions are the natural result of bringing the specified reactants and/or components together under the conditions called for pursuant to this disclosure. Thus the reactants and other materials are identified as ingredients to be brought together in connection with performing a desired chemical reaction or in forming a mixture to be used in conducting a desired reaction. Accordingly, even though the claims hereinafter may refer to substances, components and/or ingredients in the present tense ("comprises", "is", etc.), the reference is to the substance or ingredient as it existed at the time just before it was first contacted, blended or mixed with one or more other substances or ingredients in accordance with the present disclosure. The fact that the substance or ingredient may have lost its original identity through a chemical reaction or transformation or complex formation or assumption of some other chemical form during the course of such contacting, blending or mixing operations, is thus wholly immaterial for an accurate understanding and appreciation of this disclosure and the claims thereof. Nor does reference to an ingredient by chemical name or formula exclude the possibility that during the desired reaction itself an ingredient becomes transformed to one or more transitory intermediates that actually enter into or otherwise participate in the reaction. In short, no representation is made or is to be inferred that the named ingredients must participate in the reaction while in their original chemical composition, structure or form.

Each and every patent or other publication referred to in any portion of this specification is incorporated *in toto* into this disclosure by reference, as if fully set forth herein.

This invention is susceptible to considerable variation in its practice. Therefore the foregoing description is not intended to limit, and should not be construed as limiting, the invention to the particular exemplifications presented hereinabove. Rather, what is intended to be covered is as set forth in the ensuing claims and the equivalents thereof permitted as a matter of law.

## Claims

1. A process for producing a monovalent organo guanidinium salt, which comprises mixing together
(i) at least one halohydrocarbon,
(ii) at least one inorganic base, and
(iii) at least one salt of unsubstituted guanidine,
such that a product comprised predominately of organo guanidinium salt is formed.

2. A process according to Claim 1 wherein said halohydrocarbon contains from one to about 24 carbon atoms.

3. A process according to Claim 2 wherein the hydrocarbon portion of said halohydrocarbon is comprised only of carbon and hydrogen.

4. A process according to Claim 3 wherein said halohydrocarbon is a saturated halohydrocarbon.

5. A process according to Claim 4 wherein said saturated halohydrocarbon contains from one to about 12 carbon atoms.

6. A process according to Claim 5 wherein said halohydrocarbon is a monohalohydrocarbon.

7. A process according to Claim 6 wherein said halohydrocarbon is a bromohydrocarbon.

8. A process according to Claim 7 wherein said bromohydrocarbon is bromoethane.

9. A process according to any preceding claim wherein the mole ratio of halohydrocarbon to salt of unsubstituted guanidine is in the range of from about 6:1 to about 10:1.

10. A process according to any preceding claim wherein said inorganic base is an alkali metal base.

11. A process according to Claim 10 wherein said alkali metal base is an alkali metal hydroxide.

12. A process according to Claim 11 wherein said alkali metal hydroxide is sodium hydroxide.

13. A process according to any preceding claim wherein said inorganic base is an aqueous solution of the inorganic base.

14. A process according to Claim 13 wherein the concentration of inorganic base in said aqueous solution is in the range of from about 5 to about 15 moles per liter.

15. A process according to any preceding claim wherein the mole ratio of inorganic base to salt of unsubstituted guanidine is in the range of from about 7:1 to about 10:1.

16. A process according to any of Claims 1 to 5 wherein the salt of unsubstituted guanidine is selected from the group consisting of guanidine acetate, guanidine carbonate, guanidine hydrobromide, guanidine hydrochloride, guanidine nitrate, and guanidine sulfate.

17. A process according to Claim 16 wherein said salt of unsubstituted guanidine is either guanidine hydrochloride or guanidine hydrobromide.

18. A process according to Claim 17 wherein the salt of unsubstituted guanidine is guanidine hydrochloride.

19. A process according to any preceding claim wherein the temperature is in the range of 30° to 50°C.

20. A process for producing a divalent organo guanidinium salt, which comprises
(a) mixing together at least one first halohydrocarbon, at least one inorganic base, and at least one salt of unsubstituted guanidine;
(b) mixing at least one second halohydrocarbon with the mixture formed in (a), wherein said second halohydrocarbon is a dihalohydrocarbon; and
(c) mixing at least one third halohydrocarbon with the mixture formed in (b); such that a product comprised predominately of divalent organo guanidinium salt is formed.

21. A process according to Claim 20 wherein said halohydrocarbon contains from one to about 24 carbon atoms.

22. A process according to Claim 21 wherein the hydrocarbon portion of said halohydrocarbon is comprised only of carbon and hydrogen.

23. A process according to any of Claims 20 to 22 wherein said halohydrocarbon is a saturated halohydrocarbon.

24. A process according to Claim 23 wherein said saturated halohydrocarbon contains from one to about 12 carbon atoms.

25. A process according to any of Claims 20 to 24 wherein the first halohydrocarbon or third halohydrocarbon is a monohalohydrocarbon.

26. A process according to any of Claims 20 to 25 wherein said halohydrocarbon is a bromohydrocarbon.

27. A process according to Claim 26 wherein said bromohydrocarbon is bromoethane.

28. A process according to any of Claims 20 to 27 wherein said second halohydrocarbon is a bromohydrocarbon.

29. A process according to Claim 28 wherein said bromohydrocarbon is 1,2-bromoethane.

30. A process according to any of Claims 20 to 29 wherein the mole ratio of first halohydrocarbon to salt of unsubstituted guanidine is in the range of from about 4:1 to about 7:1.

31. A process according to any of Claims 20 to 30 wherein the mole ratio of second halohydrocarbon to salt of unsubstituted guanidine is in the range of from about 0.5:1 to about 1:1.

32. A process according to any of Claims 20 to 31 wherein the mole ratio of third halohydrocarbon to salt of unsubstituted guanidine is in the range of from about 1:1 to about 4:1.

33. A process according to any of Claims 20 to 32 wherein said inorganic base is an alkali metal base.

34. A process according to Claim 33 wherein said alkali metal base is an alkali metal hydroxide.

35. A process according to Claim 34 wherein said alkali metal hydroxide is sodium hydroxide.

36. A process according to any of Claim 20 to 35 wherein said inorganic base is an aqueous solution of the inorganic base.

37. A process according to Claim 36 wherein the concentration of inorganic base in said aqueous solution is in the range of from about 5 to about 15 moles per liter.

38. A process according to any of Claims 20 to 37 wherein the mole ratio of inorganic base to salt of unsubstituted guanidine is in the range of from about 7:1 to about 10:1.

39. A process according to any of Claims 20 to 38 wherein the salt of unsubstituted guanidine is selected from the group consisting of guanidine acetate, guanidine carbonate, guanidine hydrobromide, guanidine hydrochloride, guanidine nitrate, and guanidine sulfate.

40. A process according to Claim 39 wherein said salt of unsubstituted guanidine is either guanidine hydrochloride or guanidine hydrobromide.

41. A process according to Claim 40 wherein the salt of unsubstituted guanidine is guanidine hydrochloride.

42. A process according to any of Claims 20 to 41 wherein the temperature is in the range of 30° to 50°C.
